# EUROPEAN PATENT SPECIFICATION

(11) **EP 1 252 863 B1**
(45) Date of publication and mention of the grant of the patent: **06.09.2006**
(21) Application number: 02102018.5
(22) Date of filing: 20.09.1994
(51) Int. Cl.: A61B 10/00

(54) **Multiple biopsy sampling coring device**
Vorrichtung zur Mehrfach-Entnahme von Biopsie-Proben
Dispositif de biopsie permettant de prélever des échantillons multiples

(30) Priority: 20.09.1993 US 124272; 30.09.1993 US 129653; 29.10.1993 US 146447
(43) Date of publication of application: 30.10.2002
(62) Divisional of application: 94929247.8
(73) Proprietor: BOSTON SCIENTIFIC CORPORATION, Watertown, MA 02172 (US)
(72) Inventor: Robinson, Donald, MA 01748, Hopkinton (US); Banik, Michael S., OH 45241, Cincinnatti (US)
(74) Representative: Brunner, Michael John

(56) References cited:
- FR-A- 2 625 429
- US-A- 4 461 305
- US-A- 5 133 360
- US-A- 5 197 484

## Description

This invention relates to taking samples of tissue from the body for biopsy analysis.

Tissue samples can be examined in a laboratory to determine the presence of a pathological disorder (e.g. malignancy). Often, the samples must be obtained from deep within the body using a medical sampling instrument. It is usually best to obtain several samples around the location where the disorder is suspected so that the presence and progress of disease, if any, can be accurately determined. The samples must be catalogued according to the location from which each sample is taken and the integrity of the samples must be maintained for the subsequent laboratory analysis.

US 4,785,826 discloses an instrument used to gather tissue. The device retains a gathered tissue specimen by closing the end of a hollow member while the member is in the gathering position.

US-A-4461305 discloses an apparatus for automatically extracting a sample of tissue of a predetermined size and shape from a body.

US-A-5133360 discloses a device for collecting a sample of tissue from a tissue surface, comprising:
a device body having a distal end defining a forward-facing tissue receiving opening through which tissue passes when said opening is near said tissue surface,
a severing element actuatable across the width of said tissue receiving opening when tissue from said surface extends through said opening for severing said tissue from said surface, and
a storage space proximal of and adjacent said tissue receiving opening for storage of multiple tissue samples by repeatedly passing tissue through said tissue receiving opening and actuating said severing element.

Such a device is, according to the present invention, characterised in that
said severing element comprises a cutting loop, said cutting loop being preformed such that, when extended distally, said cutting loop forms an open configuration with an opening of said cutting loop being oriented generally coaxially with said tissue-receiving opening and that, when withdrawn proximally, said cutting loop forms a closed configuration in which the loop passes across said tissue receiving opening to sever said tissue.

The cutting loop may be actuated by a control wire extending parallel with the axis of said body.

The control wire may pass through a lumen extending to an opening adjacent said tissue-receiving opening.

The cutting loop may be actuated by withdrawing it into said lumen.

The device may further comprise a retractor extendable distally beyond said tissue opening and said cutting loop for engaging said tissue surface and retractable proximally to draw tissue through said tissue-receiving opening and cutting loop.

The retractor may be a spear-form element adapted to pierce tissue and having a retaining barb on its distal end.

Preferably, a cutting guide is also provided on the outer surface of the body of the device proximal of said tissue-receiving opening, said guide formed by a slot including a cutting edge with a portion communicating with the periphery of said opening such that tissue prolapses into said slot when the periphery of said device is placed against said tissue surface, said device being rotatable about its axis so that tissue in said slot is cut by said cutting edge and tissue passes through said tissue-receiving opening, the depth of said cut and the width of tissue passing through said opening being determined by the degree of rotation of said body and the width of said guide.

Preferably, the depth of said cut is 1-2 mm for each rotation of said device.

The invention has many advantages. For example, sampling into tissue to a desired depth can be achieved by controlling the amount of sample that enters the forward-facing coring opening of the device, by, for example, controlling how deeply the device is advanced into a tissue wall or how much tissue is drawn through the opening using a retractor. Thick samples can be taken in a single sampling action; for example, samples beyond the mucosal layer (submucosal sampling) can be taken from a site in a single sampling action. Careful control of sampling depth also permits samples to be taken from very thin tissue walls without puncturing the walls. Multiple samples can be taken, stored in the device, and maintained in a hydrated state without removing the device from the body.

An example of the present invention will now be described with reference to the drawings, in which:
Fig. 1 is a perspective view of an embodiment of the invention being delivered into the body through an endoscope;
Figs. 2-2e illustrate the structure and use of an embodiment of the invention;
Figs. 3-3e illustrate the structure and use of another embodiment of the invention;
Fig. 4 illustrates the structure and use of another embodiment of the invention;
Figs. 5-5c illustrate the structure and use of another embodiment of the invention.

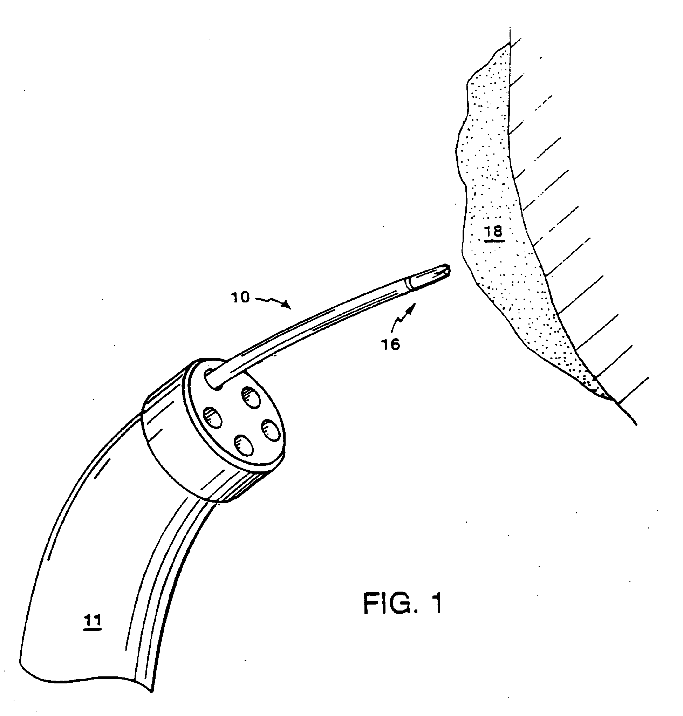

Referring to Fig. 1, the device 10 for multiple biopsy sampling may be delivered into the body through the channel of an endoscope device 11 (e.g., gastroscope, sigmoidoscope, or colonoscope). The endoscope device typically has a length of about 100-250 cm and a channel diameter of 2.0 - 3.8 mm, typically about 2.8 mm. A distal sampling portion 16 is extended from the endoscope for cutting and storing a sample of tissue from a body surface 18 of a patient (e.g. from a surface in the gastrointestinal tract or bronchial tract). The device has a diameter of preferably around 1.8 - 2.4 mm, typically about 2.3 mm or less and is of sufficient flexibility so it passes easily though the channel when the endoscope follows a tortuous body passageway. The endoscope includes other lumens for water, air, suction, and viewing. Devices according to the invention can be adapted to be introduced to sites (e.g., urinary tract, reproductive organs, cardiac tissue, or the like) deep within the body by other means. For example, a device can be configured with a lumen so that it can be advanced over a guidewire, e.g., in vascular applications. The device may be passed through an introducer or guiding catheter in, e.g., cardiac applications. The sampling and storage arrangements may be useful in open surgery applications, in breast biopsy in which the device is pressed directly into tissue, laproscopic biopsy in which the cutting element is positioned through a tubular instrument extending through the skin, and percutaneous needle biopsy in which the device is directed through a hole in the skin to sample an internal organ, e.g., the liver.

Various cutting element arrangements are possible; for example, elements that rotate about a pivot point and are biased by a spring could be provided. Arrangements with more than two cutting elements may also be used. One of either the cutting member or tubular member may be moveable and the other stationary. The components that experience sliding motion may include a lubricant. For example, the interior wall of the inner tubular member may include a low friction coating 17, e.g., of teflon, silicone, or a hydrogel, so that samples within the tube and storage space slide easily. The outer surface of the inner tubular member and/or the inner surface of the outer tubular member may also include a lubricant to ease sliding motion. (Other sliding components in other embodiments, shown below, e.g., control wires and cutting loops may also include a lubricant.)

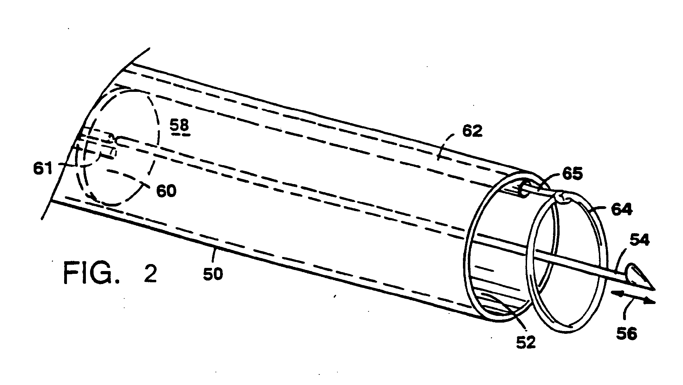

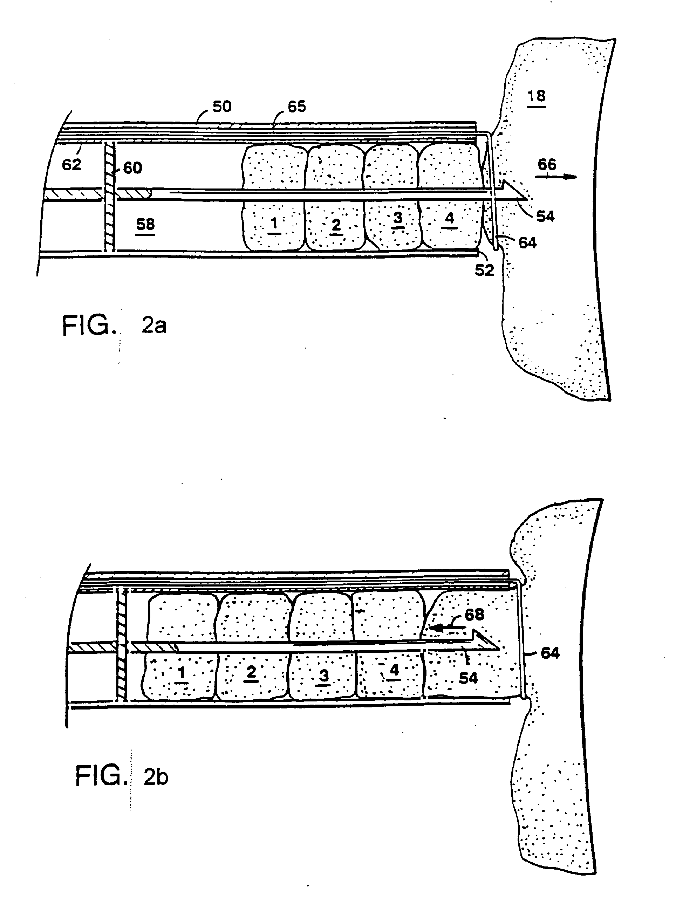

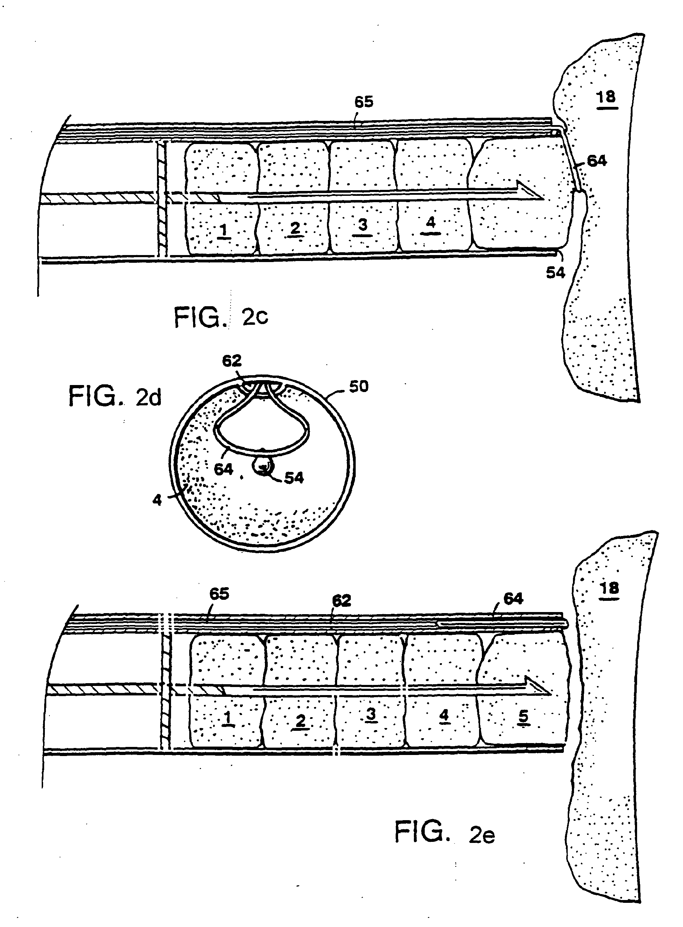

Referring to Figs. 2-2e, in this embodiment, a tubular member 50 defines at its distal end, a forward-facing distal opening 52 and includes within a retractor 54, preferably a spear-form element with a barb as shown, or another retractor type as discussed above. The retractor 54 is axially movable (arrow 56) and extends through a storage space 58 bounded on the proximal end by a sample stop 60. The axial motion of the retractor 54 and stop 60 are controlled separately; the retractor passes through an aperture in the stop and the stop is controlled by a separate member 61. (Alternatively, in other embodiments, the retractor may be attached to the stop so they move axially together.)

The embodiment also includes a wire-form cutting loop 64. The loop, shown extended in Fig. 2, is oriented with its centre roughly along the axis of the device and sized to approximate or be larger than the outer diameter of the distal portion of tubular member 50. The loop 64 may be formed of a shape memory metal, e.g., nitinol or other elastic materials, such as cold-worked stainless steel, or a plastic, that can be preformed and trained so it is capable of being repeatedly withdrawn into a lumen 62, where the loop is in a compacted state, and then extended therefrom to open and orient the loop as shown.

A control wire 65, for retracting and extending the loop, may be made integrally or attached to the loop. The lumen 62 may be constructed integrally with the tubular member 50.

Referring particularly to Fig. 2a (cross-sectional view), in use, the control wire 65 is extended distally so the cutting loop 64 is formed and positioned just distal of the open end 52 of the device 50, which is brought in proximity of the surface 18 where a sample is be taken. The retractor 54 is extended distally (arrow 66) so it pierces the tissue surface 18. As the retractor 54 is extended distally, previously taken samples, samples 1-4, are displaced proximally.

Referring particularly to Fig. 2b, the retractor 54 is then withdrawn proximally (arrow 68), bringing tissue into the distal end opening of the member 50 and through the cutting loop 64.

Referring particularly to Figs. 2c, 2d (end on view, tissue not shown) and 2e, the tissue sample is severed from the surface 18 by withdrawing the control wire 65 proximally, which draws the loop 64 through tissue across the end opening 52 of the member 50. With the loop 64 substantially withdrawn in the lumen 62, the new sample, sample 5, is completely severed from the surface 18 (Fig. 2e). The process can be repeated by extending control wire 65, distally and hence forming the cutting loop 64, to cut the next sample, as shown in Fig. 2a.

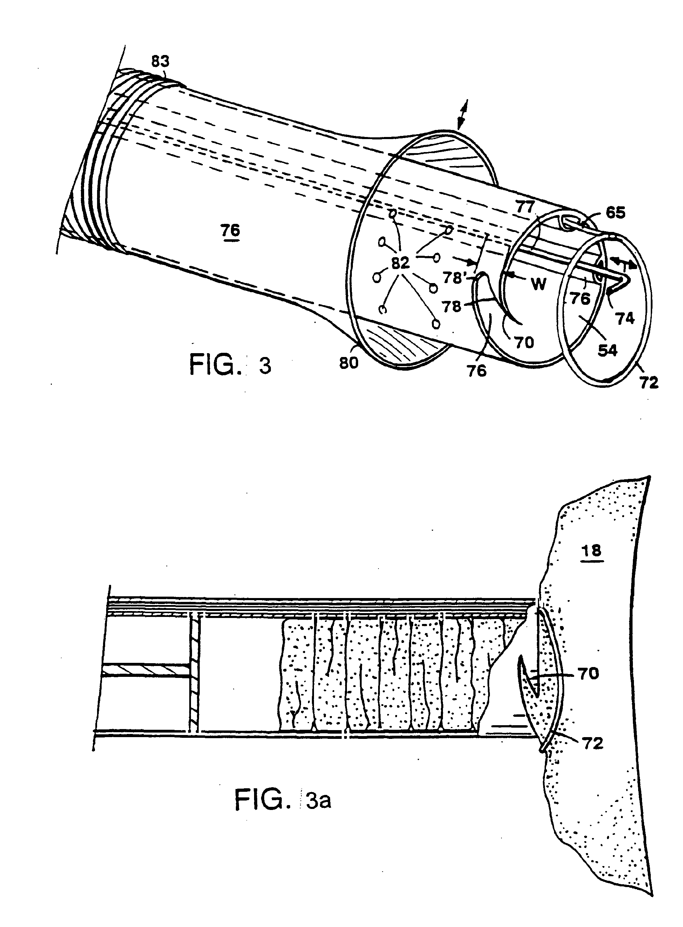

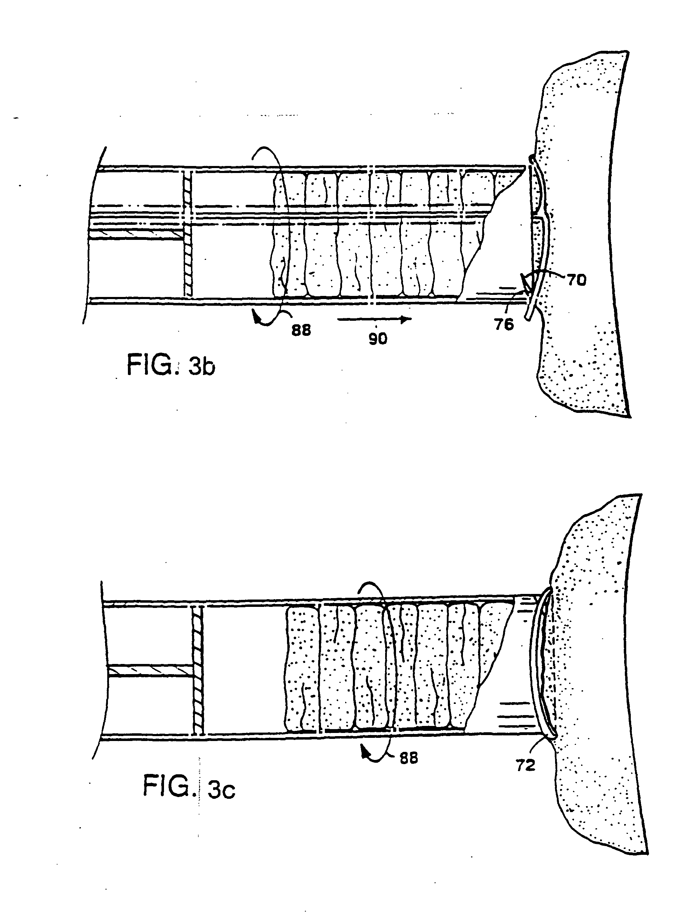

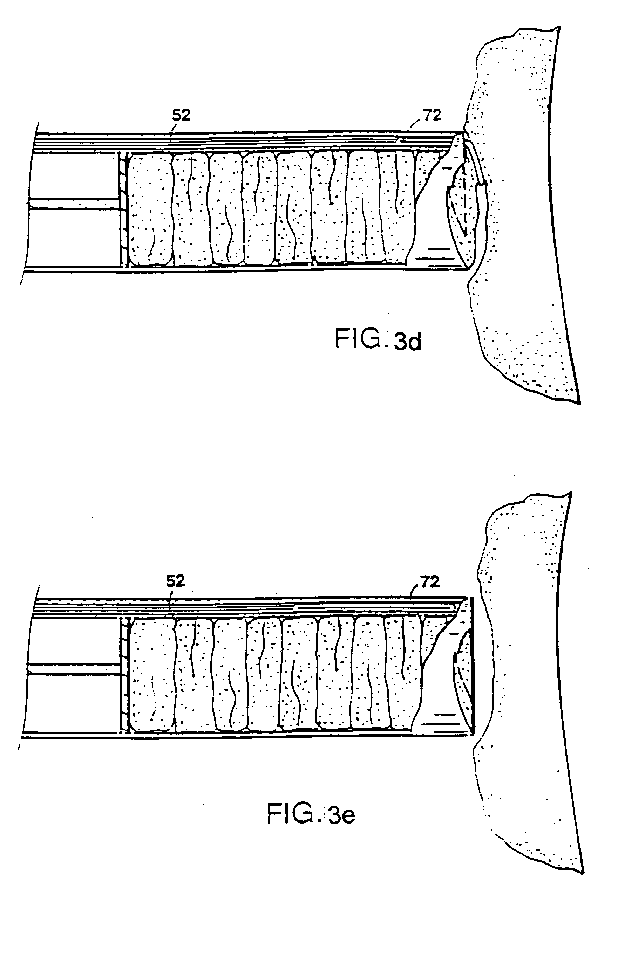

Referring to Figs. 3-3e, another embodiment is shown. In this case, the device includes near the end opening 54, a cutting guide 70, formed in the wall of the tubular body adjacent the distal end. (The guide does not extend across the diameter of the end opening; rather, it is formed by cutting out a portion of the side wall of a tube.) The device further includes a cutting loop 72, having an open diameter larger than the diameter of the end 54 of the device.

In this embodiment, the depth of the sample cut can be carefully and conveniently controlled. The end 54 of the device is pressed against the tissue wall. (The end need not be sharpened to a tissue-cutting edge.) Tissue prolapses into the gap 76 proximal of the cutting guide 70. The gap is bordered by sharpened cutting edges 78, 78*. Thus, tissue cutting occurs only during rotation of the device. The depth of tissue cut during rotation is controlled by the width of the cutting guide W from the end 54 of the device to the lowermost proximal portion of the cutting edge 78. Thus, for a single rotation, a helical, circumferential cut through tissue is made to a depth no greater than the width W. The cutting loop 72, of an enlarged diameter, slides distally over the body of the device as the distal end enters the tissue. The loop 72 can be retracted to sever the sample of controlled depth from the body surface. An axially movable retracting arm 74, passing through a slotted lumen 76, may also be provided to pull samples into the body of the device for storage. The lumen slot 77 allows the radially extending arm to be withdrawn proximally. An optional stop member 80, in this case a conical member that widens to greater diameter in the distal direction, can be positioned on the device to assure that coring beyond a predetermined depth does not occur. The stop member can be an umbrella-type assembly that changes the axial location of the end of the stop by opening and closing radially, as shown. Other stop mechanisms, such as an inflatable balloon or a spring-form wire may also be used. Apertures 82 are placed in the body of the device so that fluids (e.g. ambient body fluids) can easily pass to contact previously taken samples while they are being stored to keep them from drying out, which can damage cell structure and make pathological examination more difficult. Saline solution or the like may also be passed from proximal portions e.g. through the main lumen, into contact with samples. The saline may flow out of the apertures 82.

The body of the device is preferably formed of a highly torqueable plastic tube, but for the distal end including the cutting tang 70, which is preferably formed of metal. The proximal portions of the device include a wire coil body 83 that can be passed through torturous passageways, e.g., an endoscope channel. The shape of the guide can be varied to affect various depths and cutting profile. Typically, the most distal end of the guide terminates in a sharp point, as shown, to help start the cutting when rotation begins. The control wire and/or the cutting loop may be made of braided wires. The cutting loop may be shaped along its inner edge to form a sharp cutting surface. The cutting loop may also be heated e.g., by electric current. The end of the tubular member may flare outward, distally to facilitate drawing tissue into the end.

Referring particularly to Fig. 3a, in use, the enlarged cutting loop 72 is extended and the distal end of the device, including front-facing surface of the cutting guide 70, is pressed against the surface 18 of tissue from which a sample is be taken. Tissue prolapses into the gap 76 of the cutting guide. (This embodiment does not include retractor 74 or stop 80. The mechanical simplicity of this embodiment is an advantage, since, it can be delivered through tighter lumen tracts to hard-to reach sampling sites.)

Referring particularly to Fig. 3b, the body of the device is pressed against the tissue and rotated about its axis (arrow 88), causing tissue in the gap 76 to be cut by the sharpened edges 78, 78*.

Referring particularly to Fig.3c, as the distal end of the device enters the tissue due to the rotating cutting motion, the cutting loop 72 is pushed proximally, over the outside of the body.

Referring particularly to Fig. 3d, when the device has entered the tissue to a desired depth, for example, a single rotation of the device cutting to a depth equal to the width of the guide, a sample can be severed from the tissue surface 18 by withdrawing the cutting loop 72 into the lumen 52. The cutting loop, drawn back into the lumen 62 over the body, cuts the tissue across the opening of the distal end of the device.

Referring particularly to Fig. 3e, with the cutting loop 72 drawn substantially completely into the lumen 52, a thickness-controlled sample is severed from the tissue surface 18. The procedure above can be repeated to take an additional sample if desired.

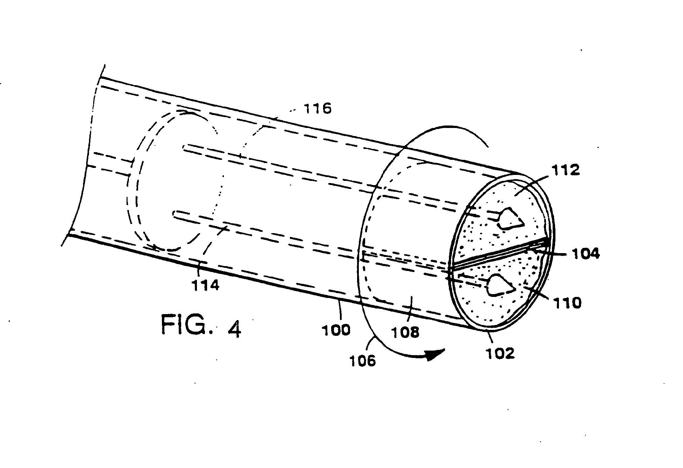

Referring to Fig. 4, another embodiment is shown. In this case, the device includes a tubular member 100, having an open tubular distal end 102. The distal end 102 is sharpened so the device may be urged into tissue to a desired depth, by extending the device distally axially. A cutting wire 104 is provided across the circumference of the device. To sever a tissue sample, the body of the device is rotated about its axis (arrow 106). For each sample taken, the device produces two sample halves. For example, sample halves 110, 112 of sample 108, since the tissue has been bisected by the cutting wire 104 when the device was urged distally into the tissue surface. The device may further be provided with retractor members 114, 116, which may be in the form of a spear-form element or other retractors as described above.

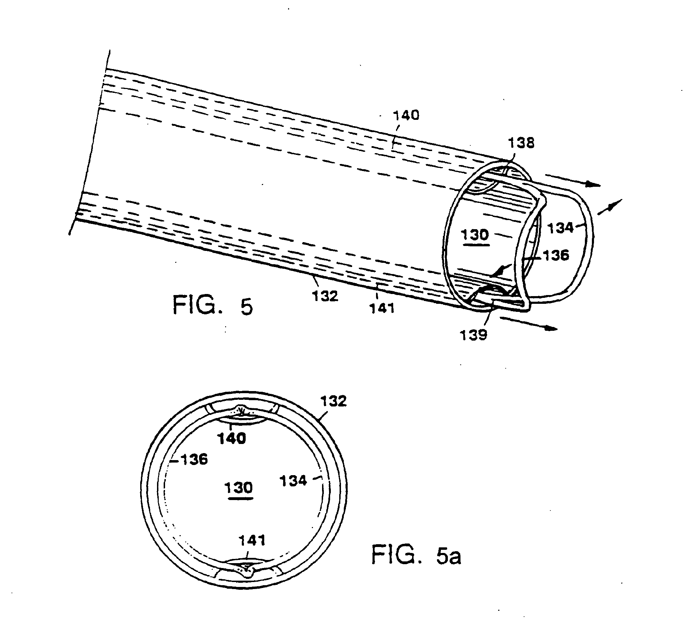

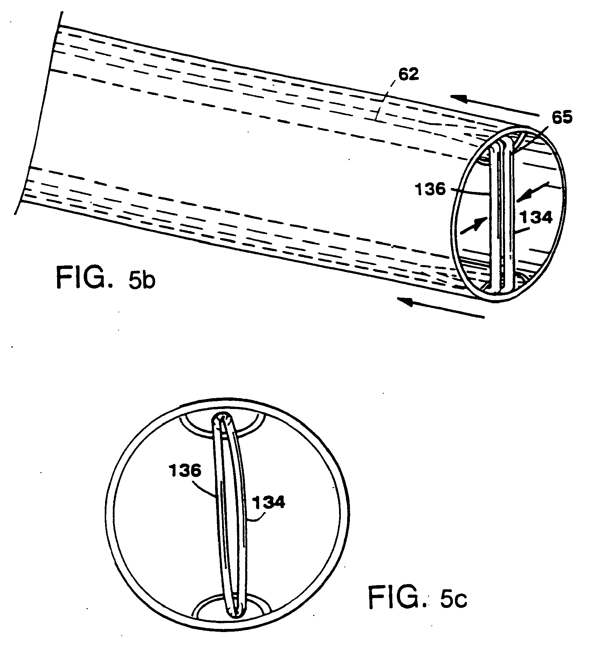

Referring to Figs. 5-5c, another embodiment for severing tissue is shown. In this case, an arrangement for severing tissue across the distal end 130 of a tube 132 includes a pair of wire cutting elements 134, 136, that join at respective ends to control wires 138, 139, that are axially moveable in lumens 140, 141. In the configuration shown in Figs. 5 and 5a (end-on view), the control wires are extended, which bows the wire cutting elements 134, 136 outward, causing them to conform to the outer end of the tubular member so that tissue can extend beyond the wires and enter the distal end of the tube. In the configuration of Figs. 5b and 5c, the control wires are drawn proximally, causing the cutting elements 134, 136 to close across the end opening of the tube, cutting tissue so that a sample can be severed from a tissue surface. In other embodiments, a single control wire is used; the other ends of the cutting elements are attached to the end of the tube.

The features discussed above with respect to the various embodiments, e.g. arrangements for receiving tissue through the opening of the devices and arrangements for severing tissue from a surface, can be combined in further embodiments.

## Claims

1. A device for collecting a sample of tissue from a tissue surface (18), comprising:
a device body (50) having a distal end defining a forward-facing tissue receiving opening (52) through which tissue (1-5) passes when said opening (52) is near said tissue surface (18),
a severing element (64,72) actuatable across the width of said tissue receiving opening (52) when tissue (1-5) from said surface (18) extends through said opening (52) for severing said tissue (1-5) from said surface (18), and
a storage space (58) proximal of and adjacent said tissue receiving opening (52) for storage of multiple tissue samples (1-5) by repeatedly passing tissue (1-5) through said tissue receiving opening (52) and actuating said severing element (64,72),
**characterised in that**
said severing element (64,72) comprises a cutting loop (64,72), said cutting loop (64,72) being preformed such that, when extending distally, said cutting loop (64,72) forms an open configuration with an opening of said cutting loop (64,72) being oriented generally coaxially with said tissue-receiving opening and that, when withdrawn proximally, said cutting loop (64,72) forms a closed configuration in which the loop passes across said tissue receiving opening (52) to sever said tissue (1-5).

2. The device of claim 1, wherein said cutting loop (64,72) is actuated by a control wire (65) extending parallel with the axis of said body (50).

3. The device of claim 2, wherein said control wire (65) passes through a lumen (62) extending to an opening adjacent said tissue-receiving opening (52).

4. The device of any of claims 1 to 3, wherein said cutting loop (64,72) is actuated by withdrawing it into said lumen (62).

5. The device of any of claims 1 to 4, further comprising a retractor (54,74) extendable distally beyond said tissue receiving opening (52) and said cutting loop (64,72) for engaging said tissue surface (18) and retractable proximally to draw tissue (1-5) through said tissue receiving opening and cutting loop (64,72).

6. The device of claim 5, wherein said retractor (54) is a spear-form element adapted to pierce tissue and having a retaining barb on its distal end.

7. The device of any of claims 1 to 6, further including a cutting guide (70) on the outer surface of the device body (50) proximal of said tissue receiving opening (52), said guide (70) formed by a slot including a cutting edge with a portion communicating with the periphery of said tissue receiving opening such that tissue prolapses into said slot when the periphery of said device body (50) is placed against said tissue surface,
said device body (50) being rotatable about its axis so that tissue in said slot is cut by said cutting edge and tissue passes through said tissue receiving opening (52), the depth of said cut and the width of tissue passing through said tissue receiving opening being determined by the degree of rotation of said device body and the width of said guide.

8. The device of claim 7, wherein the depth of said cut is 1-2 mm for each rotation of said device body (50).

9. The device of claim 1, wherein the cutting loop (64,72) is sized to approximate or be larger than an outer diameter of the distal end of the device body.

10. The device of claim 1, wherein the cutting loop (64,72) is made of a shape memory material.

11. The device of claim 1, further comprising a sample stop (60) disposed inside the storage space (58).

## Patentansprüche

1. Vorrichtung zum Sammeln einer Gewebeprobe von einer Gewebeoberfläche (18), aufweisend:
einen Vorrichtungskörper (50) mit einem distalen Ende, welches eine nach vom gerichtete Gewebeaufnahmeöffnung (52) definiert, durch welche das Gewebe (1-5) passiert, wenn die Öffnung (52) sich in der Nähe der Gewebeoberfläche (18) befindet,
ein Trennelement (64, 72) betätigbar über die Breite der Gewebeaufnahmeöffnung (52), wenn Gewebe (1-5) von der Oberfläche (18) sich durch die Öffnung (52) erstreckt, zum Teilen des Gewebes (1-5) von der Oberfläche (18), und
einen Aufbewahrungsraum (58), der proximal zu der und angrenzend an die Gewebeaufnahmeöffnung (52) ist, für eine Aufbewahrung von mehreren Gewebeproben (1-5) durch wiederholendes Passieren von Gewebe (1-5) durch die Gewebeaufnahmeöffnung (52) und Betätigen des Trennelementes (64, 72),
**dadurch gekennzeichnet, dass**
das Trennelement (64, 72) einen Schneidering (64, 72) umfasst, wobei der Schneidering (64, 72) ausgeführt ist, so dass, wenn er sich distal erstreckt, der Schneidering (64, 72) eine offene Stellung bildet mit einer Öffnung des Schneiderings (64, 72), welche üblicherweise koaxial orientiert ist mit der Gewebeaufnahmeöffnung, und dass, wenn proximal zurückgezogen, der Schneidering (64, 72) eine geschlossene Stellung bildet, in der der Ring über die Gewebeaufnahmeöffnung (52) passiert, zum Trennen des Gewebes (1-5).

2. Vorrichtung nach Anspruch 1, bei welcher der Schneidering (64, 72) betätigt wird durch einen Steuerdraht (65), welcher sich parallel mit der Achse des Körpers (50) erstreckt.

3. Vorrichtung nach Anspruch 2, bei welcher der Steuerdraht (65) durch ein Lumen (62) passiert, welches sich zu einer Öffnung erstreckt, welche angrenzend an die Gewebeaufnahmeöffnung (52) ist.

4. Vorrichtung nach irgendeinem der Ansprüche 1 bis 3, bei welcher der Schneidering (64, 72) betätigt wird durch Zurückziehen desselben in das Lumen (62).

5. Vorrichtung nach irgendeinem der Ansprüche 1 bis 4, weiter aufweisend einen Retraktor (54, 74), welcher distal verlängerbar ist über die Gewebeaufnahmeöffnung (52) und den Schneidering (64, 72) hinaus für einen Eingriff mit der Gewebeoberfläche (18) und welcher proximal zurückziehbar ist zum Herausziehen von Gewebe (1-5) durch die Gewebeaufnahmeöffnung und den Schneidering (64, 72).

6. Vorrichtung nach Anspruch 5, bei welcher der Retraktor (54) ein speerartiges Element ist, welches geeignet ist zum Durchstechen von Gewebe und mit einem Haltewiderhaken auf seinem distalen Ende.

7. Vorrichtung nach irgendeinem der Ansprüche 1 bis 6, weiter umfassend eine Schneideführung (70) auf der äußeren Oberfläche des Vorrichtungskörpers (50) proximal zu der Gewebeaufnahmeöffnung (52), wobei die Führung (70) durch einen Schlitz gebildet wird, welcher eine Schneidekante umfasst mit einem Abschnitt, welcher mit der Peripherie der Gewebeaufnahmeöffnung in Verbindung steht, so dass Gewebe in den Schlitz prolabiert, wenn die Peripherie des Vorrichtungskörpers (50) gegen die Gewebeoberfläche platziert wird,
wobei der Vorrichtungskörper (50) drehbar ist um seine Achse, so dass Gewebe in dem Schlitz geschnitten wird von der Schneidekante und Gewebe durch die Gewebeaufnahmeöffnung (52) passiert, wobei die Tiefe des Schnittes und die Breite von Gewebe, welches durch die Gewebeaufnahmeöffnung passiert, bestimmt wird durch den Rotationsgrad des Vorrichtungskörpers und die Breite der Führung.

8. Vorrichtung nach Anspruch 7, bei welcher die Tiefe der Schnittes 1-2 mm ist für jede Rotation des Vorrichtungskörpers (50).

9. Vorrichtung nach Anspruch 1, bei welcher der Schneidering (64, 72) bemessen ist annähernd gleich oder größer zu sein als ein äußerer Durchmesser des distalen Endes des Vorrichtungskörpers.

10. Vorrichtung nach Anspruch 1, bei welcher der Schneidering (64, 72) aus einem Formgedächtnismaterial hergestellt ist.

11. Vorrichtung nach Anspruch 1, weiter umfassend einen Entnahmeaufhalter (60), welcher innerhalb des Aufbewahrungsraums (58) angeordnet ist.

## Revendications

1. Dispositif pour recueillir un échantillon de tissu à partir d'une surface de tissu (18), comprenant :
un corps de dispositif (50) ayant une extrémité distale définissant une ouverture frontale de réception de tissu (52) par laquelle passe le tissu (1-5) lorsque ladite ouverture (52) est proche de ladite surface de tissu (18),
un élément de sectionnement (64,72) pouvant être actionné sur toute la largeur de ladite ouverture de réception de tissu (52) lorsque le tissu (1-5) à partir de ladite surface (18) passe à travers ladite ouverture (52) pour le sectionnement dudit tissu (1-5) à partir de ladite surface (18), et
un espace de stockage (58) proximal et contigu à ladite ouverture de réception de tissu (52) pour le stockage de multiples échantillons de tissu (1-5) en faisant passer de façon répétée le tissu (1-5) à travers ladite ouverture de réception de tissu (52) et en actionnant ledit élément de sectionnement (64,72),
**caractérisé en ce que**,
ledit élément de sectionnement (64,72) comprend une boucle de coupe (64,72), ladite boucle de coupe (64,72) étant préformée de telle sorte que lorsqu'elle sort distalement, ladite boucle de coupe (64,72) forme une configuration ouverte avec une ouverture de ladite boucle de coupe (64,72) qui est orientée de façon générale coaxialement par rapport à ladite ouverture de réception de tissu et **en ce que**, lorsqu'elle est rentrée proximalement, ladite boucle de coupe (64,72) forme une configuration fermée, dans laquelle la boucle passe par ladite ouverture de réception de tissu (52) pour sectionner ledit tissu (1-5).

2. Dispositif selon la revendication 1, dans lequel ladite boucle de coupe (64,72) est actionnée par un fil de commande (65) s'étendant parallèlement à l'axe dudit corps (50).

3. Dispositif selon la revendication 2, dans lequel ledit fil de commande (65) traverse un orifice (62) s'étendant jusqu'à une ouverture contiguë à ladite ouverture de réception de tissu (52).

4. Dispositif selon l'une quelconque des revendications 1 à 3, dans lequel ladite boucle de coupe (64,72) est actionnée pour sa rentrée dans ledit orifice (62).

5. Dispositif selon l'une quelconque des revendications 1 à 4, comprenant de plus un rétracteur (54,74), pouvant s'étendre distalement au-delà de ladite ouverture de réception de tissu (52) et de ladite boucle de coupe (64,72) pour l'engagement de ladite surface de tissu (18) et rétractable proximalement pour tirer le tissu (1-5) à travers ladite ouverture de réception de tissu et la boucle de coupe (64,72).

6. Dispositif selon la revendication 5, dans lequel ledit rétracteur (54) est un élément en forme de pointe de lance capable de perforer le tissu et comportant un picot de retenue sur son extrémité distale.

7. Dispositif selon l'une quelconque des revendications 1 à 6, comprenant de plus un guidage de coupe (70) sur la surface extérieure du corps de dispositif (50) proximale de ladite ouverture de réception de tissu (52), ledit guidage (70) étant formé par une fente incorporant un bord de coupe avec une portion communiquant avec la périphérie de ladite ouverture de réception de tissu de telle sorte que le tissu tombe dans ladite fente, lorsque la périphérie dudit corps de dispositif (50) est placée contre ladite surface de tissu,
ledit corps de dispositif (50) étant rotatif autour de son axe, de sorte que le tissu dans ladite fente est coupé par ledit bord de coupe et le tissu traverse ladite ouverture de réception de tissu (52), la profondeur de ladite coupe et la largeur du tissu traversant ladite ouverture de réception de tissu étant déterminées par le degré de rotation dudit corps de dispositif et la largeur dudit guidage.

8. Dispositif selon la revendication 7, dans lequel la profondeur de ladite coupe est de 1-2mm à chaque rotation dudit corps de dispositif (50).

9. Dispositif selon la revendication 1, dans lequel la boucle de coupe (64,72) est dimensionnée de façon à être approximativement égale ou supérieure à un diamètre extérieur de l'extrémité distale du corps de dispositif.

10. Dispositif selon la revendication 1, dans lequel la boucle de coupe (64,72) est réalisée à partir d'un matériau à mémoire de forme.

11. Dispositif selon la revendication 1, comprenant de plus une butée d'échantillon (60) disposée à l'intérieur de l'espace de stockage (58).
